(19) Europäisches Patentamt / European Patent Office / Office européen des brevets

(11) **EP 3 654 828 B1**

(12) # EUROPEAN PATENT SPECIFICATION

(45) Date of publication and mention of the grant of the patent:
**10.01.2024 Bulletin 2024/02**

(21) Application number: **18742497.3**

(22) Date of filing: **20.07.2018**

(51) International Patent Classification (IPC):
***A61B 5/00*** *(2006.01)*

(52) Cooperative Patent Classification (CPC):
**A61B 5/0095; A61B 5/0062; A61B 5/7207;**
A61B 5/0073; A61B 5/14546; A61B 5/14551;
A61B 5/441; A61B 5/489; A61B 5/7246;
A61B 2576/00; G16H 30/40

(86) International application number:
**PCT/EP2018/069736**

(87) International publication number:
**WO 2019/016361 (24.01.2019 Gazette 2019/04)**

(54) **SYSTEM FOR OPTOACOUSTIC IMAGING, IN PARTICULAR FOR RASTER-SCAN OPTOACOUSTIC MESOSCOPY, AND METHOD FOR OPTOACOUSTIC IMAGING DATA PROCESSING**

SYSTEM ZUR OPTOAKUSTISCHEN BILDGEBUNG, INSBESONDERE ZUR OPTOAKUSTISCHEN RASTERSCAN-MESOSKOPIE, UND VERFAHREN ZUR VERARBEITUNG VON OPTOAKUSTISCHEN BILDGEBUNGSDATEN

SYSTÈME D'IMAGERIE OPTOACOUSTIQUE, EN PARTICULIER POUR MÉSOSCOPIE OPTOACOUSTIQUE À BALAYAGE DE TRAME, ET PROCÉDÉ DE TRAITEMENT DE DONNÉES D'IMAGERIE OPTOACOUSTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.07.2017 EP 17182595**

(43) Date of publication of application:
**27.05.2020 Bulletin 2020/22**

(73) Proprietor: **Helmholtz Zentrum München Deutsches Forschungszentrum für Gesundheit und Umwelt (GmbH)**
**85764 Neuherberg (DE)**

(72) Inventors:
• **NTZIACHRISTOS, Vasilis**
  **80939 München (DE)**
• **AGUIRRE BUENO, Juan**
  **28043 Madrid (ES)**
• **SCHWARZ, Mathias**
  **81379 München (DE)**

(74) Representative: **Linsmeier, Josef**
**Wallinger Ricker Schlotter Tostmann**
**Patent- und Rechtsanwälte Partnerschaft mbB**
**Zweibrückenstrasse 5-7**
**80331 München (DE)**

(56) References cited:
**US-A1- 2016 038 021**

• **SOLIMAN DOMINIK ET AL: "Combined label-free optical and optoacoustic imaging of model organisms at mesoscopy and microscopy resolutions", PROGRESS IN BIOMEDICAL OPTICS AND IMAGING, SPIE - INTERNATIONAL SOCIETY FOR OPTICAL ENGINEERING, BELLINGHAM, WA, US, vol. 9708, 15 March 2016 (2016-03-15), pages 97083B-97083B, XP060064658, ISSN: 1605-7422, DOI: 10.1117/12.2208861 ISBN: 978-1-5106-0027-0**

- SCHWARZ MATHIAS ET AL: "Visualization of the microcirculatory network in skin by high frequency optoacoustic mesoscopy", PROGRESS IN BIOMEDICAL OPTICS AND IMAGING, SPIE - INTERNATIONAL SOCIETY FOR OPTICAL ENGINEERING, BELLINGHAM, WA, US, vol. 9539, 16 July 2015 (2015-07-16), pages 95390J-95390J, XP060056425, ISSN: 1605-7422, DOI: 10.1117/12.2183463 ISBN: 978-1-5106-0027-0
- Bhavna Antony ET AL: "Automated 3-D method for the correction of axial artifacts in spectral-domain optical coherence tomography images", Biomedical Optics Express, 1 August 2011 (2011-08-01), pages 2403-2416, XP055518719, United States DOI: 10.1364/BOE.2.002403 Retrieved from the Internet: URL:https://www.ncbi.nlm.nih.gov/pmc/articles/PMC3149538/pdf/2403.pdf
- YIH MIIN LIEW ET AL: "Motion correction of in vivo three-dimensional optical coherence tomography of human skin using a fiducial marker", BIOMEDICAL OPTICS EXPRESS, vol. 3, no. 8, 1 August 2012 (2012-08-01), page 1774, XP055518828, United States ISSN: 2156-7085, DOI: 10.1364/BOE.3.001774
- Mathias Schwarz: "Multispectral Optoacoustic Dermoscopy: Methods and Applications", Dissertation, 21 November 2017 (2017-11-21), pages 1-141, XP055532444, DE Retrieved from the Internet: URL:https://mediatum.ub.tum.de/doc/1324031/1324031.pdf

**Description**

The present invention relates to a system for optoacoustic imaging, in particular for raster-scan optoacoustic mesoscopy, and an according method for optoacoustic imaging data processing according to the independent claims.

Optoacoustic imaging is based on the photoacoustic effect, according to which ultrasound waves are generated due to absorption of transient electromagnetic radiation by an object, e.g. a biological tissue, and a subsequent thermoelastic expansion of the object. The ultrasound waves are detected and converted into electrical signals, based on which an image can be reconstructed.

Raster-scan optoacoustic mesoscopy (RSOM), also termed photoacoustic mesoscopy, is a biomedical optical imaging technique capable of visualizing tissue morphology, e.g. vascular morphology, and pathophysiological biomarkers of inflammatory diseases through several millimeters of depth while preserving high resolution, typically in the 5 to 20 micron range. By using ultra-wideband ultrasound detectors RSOM allows for label-free imaging and quantification of inflammatory biomarkers in psoriasis and eczema. Moreover, multi-wavelength illumination and spectral unmixing allows for visualization of a larger number of pathophysiological features, including tissue/blood oxygenation, melanin patterns, or the bio-distribution of externally administered photo-absorbing labels.

The scientific articles titled "Combined label-free optical and optoacoustic imaging of model organisms at mesoscopy and microscopy resolutions" (DOI: 10.1117/12.2208861, SCHWARZ ET AL) and "Visualization of the microcirculatory network in skin by high frequency optoacoustic mesoscopy" (DOI: 10.1117/12.2183463, BAGHERINIA ET AL) disclose system for optoacoustic imaging with Raster scan of A-scans.

The patent application US 2016/038021 A1 discloses Optical Coherence Tomography systems with corrections against axial displacements artifacts.

RSOM image formation is achieved by raster-scanning a focused ultrasound detector over the region of interest (ROI) to collect optoacoustic waves generated in the tissue in response to pulsed laser illumination. Preferably, the focal point of the ultrasound detector lies slightly above the surface of the sample, and the detector collects ultrasound signals over an acceptance angle (aperture) defined by the area and focusing characteristics of the detecting element. The collected data are tomographically reconstructed to yield an image of light absorbers within the tissue, e.g. the skin. Data acquisition speed is determined by the pulse repetition rate of the laser. Generating a two-dimensional skin scan based on a grid of 250 x 250 points may require approx. 60 seconds when the laser has a pulse repetition rate (PRR) of 1 kHz; this scan time is longer when larger grids, multi-wavelength illumination, lower laser safety limits or data averaging are used. During this time, the subject's movement, e.g. breathing motion at a respiratory rate of up to 30 times per minute, can alter the position of the ultrasound detector relative to the sample which may affect RSOM image quality. Restricting animal or human motion during RSOM scanning is challenging. For example, the RSOM scan head may be attached to the ROI using suction or adhesive tape restricting lateral movement of the ROI. Nevertheless, motion effects are not entirely eliminated since the skin surface can possibly move relative to the acoustic axis of the detector because of the use of water or acoustic gel for ultrasound coupling as well as the use of thin, acoustically transparent membranes, which create an interface between skin and coupling media.

It is, therefore, desirable to reduce the effects of such motion on RSOM imaging performance. For example, to correct for macroscopic motion in small animals, thorax cross-sections may be clustered based on the cardiac cycle. Clustering can also be employed to correct for motion artifacts due to the breathing cycle. However, these approaches are best suited to two-dimensional optoacoustic reconstructions following a single laser pulse. RSOM, however, usually involves tomographic (three-dimensional) reconstruction of an image based on a set of recorded time signals. Therefore, RSOM uses data recorded over several seconds which may be affected by motion.

It is an object of the invention to provide a system for optoacoustic imaging, in particular for raster-scan optoacoustic mesoscopy, and method for optoacoustic imaging data processing which allows for a correction and/or elimination of effects caused by motion of the object.

The object is achieved by the system and method according to the independent claims.

A system for optoacoustic imaging according to an aspect of the invention comprises: an irradiation unit configured to irradiate an object comprising biological tissue with electromagnetic radiation, a detection unit configured to detect acoustic waves generated in the object at a plurality of locations along an axial dimension and along at least one lateral dimension, which is perpendicular to the axial dimension, in response to irradiating the object with electromagnetic radiation and to generate a plurality of according detection signals, and a processing unit configured to determine an axial displacement of the course of one or more first detection signals along the axial dimension relative to the course of one or more second detection signals along the axial dimension, to correct the detection signals by reducing and/or eliminating the axial displacement, and to reconstruct at least one image of the object based on the corrected detection signals.

A method for optoacoustic imaging data processing according to another aspect of the invention comprises the following steps: retrieving a plurality of detection signals representing acoustic waves generated in an object at a plurality of locations along an axial dimension and along at least one lateral dimension, which is perpendicular to the axial dimension, in response to irradiating the object with electromagnetic radiation, determining an axial displacement of the course of one or more first detection signals along the axial dimension relative to the course of one or more second detection signals along the axial dimension, correcting the detection signals by reducing and/or eliminating the axial displacement, and reconstructing at least one image of the object based on the corrected detection signals.

A system for optoacoustic imaging according to yet another aspect of the invention comprises: an irradiation unit configured to irradiate an object comprising biological tissue with electromagnetic radiation, a detection unit located at coordinates (x, y, z) above the object and configured to detect acoustic waves generated in the object at a plurality of locations in response to irradiating the object with electromagnetic radiation and to generate a plurality of according detection signals (I(x, y, z, t)) that depend on time (t), and a processing unit configured

- to determine an axial displacement ($\Delta z$) of the imaged object due to motion by characterizing and/or determining at least one offset ($\Delta t$) of the time course of one or more first detection signals relative to the time course of one or more second detection signals, and
- to correct the detection signals by reducing and/or eliminating the at least one offset ($\Delta t$) and to reconstruct at least one image of the object based on the corrected detection signals, and/or
- to reconstruct at least one image of the object by considering the axial displacement ($\Delta z$) of the object.

An aspect of the invention is based on the approach to correct the detection signals by considering disruptions of the ultrasound wave front due to an axial displacement, which usually corresponds to a vertical displacement, of the imaged object during data acquisition by capturing and quantifying the extent or height of the disruptions contained and/or represented in the acquired two- or three-dimensional set of detection signals, which is also referred to as sinogram. The determined extent or height of the disruptions is used to correct for object movement in the axial dimension of the ultrasound wave detector, also referred to as transducer, during data acquisition. In this way, motion artifacts are reduced and RSOM resolution in the final images is improved significantly.

In particular, an offset of the detection signals along the axial dimension, also referred to as "A-lines", relative to each other is determined. To this end, a displacement of the time course or spatial course of one or more first detection signals along the axial dimension relative to the time course or spatial course of one or more second detection signals along the axial dimension is determined. Within the meaning of present invention, the time course or spatial course of a detection signal along the axial dimension or the time course or spatial course of an A-line corresponds to the course of the magnitude of a detection signal corresponding to acoustic waves generated at a plurality of different points of time $t_k$ or locations $z_k$ in the object along the axial dimension at a fixed location $x_i$, $y_j$ with respect to the lateral dimensions in the object. Preferably, the first and/or second detection signals or A-lines may be neighboring detection signals or neighboring A-lines, respectively, or neighboring sets of detection signals or neighboring sets of A-lines, respectively.

Because the time course or spatial course of the acquired detection signals along the axial dimension or A-lines contains information about the presence and/or properties of light-absorbing components of the object along the axial dimension, a possible displacement of detection signals or A-lines along the axial dimension can be determined just by considering the information contained in the detection signals along the axial dimension or in the A-lines, respectively.

Based on the determined axial displacement of detection signals along the axial dimension or A-lines relative to each other, the detection signals are corrected by reducing and/or eliminating the axial displacement, and a two- or three-dimensional image of the object is reconstructed based on the corrected detection signals.

In summary, the invention allows for a correction and/or elimination of effects caused by motion of the object in a simple and reliable way.

It is noted that the time axis (t) is not completely identical to the axial dimension (z). Rather, one-dimensional time-dependent detection signals (also referred to as "A-scans") are recorded at each lateral scan point (x, y) by collecting ultrasound waves generated within the cone-shaped sensitivity field of the focused detector, and subsequently converted into detection signals corresponding to acoustic waves generated at a plurality of locations in x, y and z dimension. For sake of simplification, however, time axis (t) and axial dimension (z) are used synonymously, unless otherwise stated. Likewise, the term "axial displacement", which preferably relates to a displacement ($\Delta z$) of the imaged object in the z dimension, is used synonymously with the term "offset of the time course" ($\Delta t$) of first and second detection signals, i.e. first and second A-lines, relative to each other.

According to a preferred embodiment of the invention, the processing unit is configured to detect a structure contained in the detection signals, the detected structure corresponding to a representation of a component of the object in the detection signals, and to determine the axial displacement of the course of the first detection signals relative to the course of the second detection signals based on an axial offset of a section of the detected structure relative to an expected structure, which corresponds to the component of the object, along the axial dimension. For example, the structure contained in the detection signals corresponds to a layer contained in the object. In case that the object has temporarily moved in axial dimension during the detection of acoustic waves, a section of the structure (i.e. a section of the representation of the layer) contained in a first set of A-lines, which was acquired during the temporary movement of the object, is axially offset relative to the remaining structure contained in a second set of A-lines, i.e. in the other A-lines, which was acquired when the object did not move during the acquisition. Assuming that the component of the object exhibits a continuous and/or smooth shape which extends more or less perpendicularly to the axial dimension and/or along the at least one lateral dimension, the expected structure contained in the detection signals would be an accordingly continuous and/or smooth structure contained in the A-lines. By determining the axial offset of the detected section of the structure relative to the expected structure, the axial displacement of the course of the first detection signals relative to the course of the second detection signals can be determined in a simple and reliable way.

Preferably, the detected structure corresponds to a representation of an endogenous component of the tissue. Preferably, the endogenous component of the tissue is at least one of the following: at least one melanin-containing layer of skin tissue, the basal layer (stratum basale) of the epidermis of skin tissue, the cornified layer (stratum corneum) of the epidermis of skin tissue, a layer of blood vessels of the tissue. In this way, an axial movement during image acquisition can be easily determined and reliably compensated just by considering information contained in the detection signals relating to inherent components of the biological tissue to be imaged, so that an application of further light-absorbing components to the tissue is dispensable.

Alternatively or additionally, the detected structure corresponds to a representation of an exogenous layer configured to absorb at least a part of the electromagnetic radiation. Preferably, the exogenous layer corresponds to a coupling element arranged between the tissue and the detection unit and is configured to acoustically couple the detection unit with the tissue and/or a medium applied to the surface of the tissue, in particular to the skin. In this way, an artificially created light-absorbing layer can be used for determining and compensating the effect of axial movement during image acquisition. The exogenous component or layer can be, e.g., an absorbing structure within the coupling element, e.g. a coupling membrane, and/or a light-absorbing layer of paint, color, spray, cream etc. applied to the tissue surface or skin. Because light-absorbing properties and/or the spatial course of such an exogenous component are known and/or can be defined as required or desired, an axial movement during image acquisition can be particularly reliably determined and compensated by considering the information contained in the detection signals relating to the exogenous component.

Preferably, the processing unit is configured to enhance and/or detect the structure contained in the detection signals by applying a bandpass filter to the detection signals and/or a segmentation of information contained in the detection signals. In this way, the structure can be detected with particularly high reliability so that the reliability of the correction of motion effects to the final images is further enhanced.

It is further preferred that the processing unit is configured to determine the section of the detected structure, which is offset relative to the expected structure along the axial dimension, based on a, preferably rough, segmentation of information contained in the detection signals along the axial dimension and along at least one lateral dimension. In this way, the structure and/or boundaries of the structure contained in the detection signals can be located and/or extracted based on information relating to the component of the object, e.g. the course of a melanin layer in the skin, represented in the detection signals.

It is also preferred that the processing unit is configured to determine a map of the detected structure along the at least one lateral dimension and to determine a map of the expected structure along the at least one lateral dimension based on the map of the detected structure. The term "map" preferably relates to a two- or three-dimensional course of the magnitude of the detection signals corresponding to acoustic waves generated at a plurality of different points of time or locations in the object along the axial dimension and different locations with respect to the at least one lateral dimension in the object. By determining the map of the expected structure based on the map of the detected structure, the expected structure can be easily determined from the detection signals without the need of further information input regarding absorption and/or structural properties of the component of the object.

Preferably, the processing unit is configured to determine the map of the expected structure by smoothing the map of the detected structure. Preferably, the processing unit is configured to smooth the map of the detected structure by applying a median filter and/or a moving average filter to the map of the detected structure along at least one lateral dimension. Smoothing the map of the detected structure is a particularly reliable and simple way to obtain the map of the expected structure.

It is, moreover, preferred that the processing unit is configured to determine the axial offset of the section of the detected structure relative to the expected structure along the axial dimension based on the map of the detected structure and the map of the expected structure. This is a particularly reliable way to determine the axial offset of the section of the detected structure contained in the detection signals relative to the expected structure which corresponds to the component of the object.

In summary, the preferred embodiments described above, which are of particular advantage in dermoscopy applications, are based on the approach to consider disruptions of the ultrasound wave front generated by a vertical or axial movement of an exogenous or endogenous component of the object, e.g. the melanin layer in the skin surface, relative to the ultrasound detector. From the disrupted skin surface, a smooth synthetic surface is generated, and the offset between the two surfaces, i.e. the disrupted skin surface and the synthetic surface, is used to correct for the relative position change of the ultrasound detector during data acquisition. In other words: By tracking the skin surface based on melanin content at each scan point, a synthetic surface, which is representative of skin motion, is determined. The synthetic surface is used to correct for sample movement in the axial dimension of the ultrasound detector during data acquisition. In this way, motion artifacts in the images are significantly reduced and/or eliminated, and a resolution in the final images up to 5-fold higher than before correction can be achieved.

According to another preferred embodiment of the invention, the processing unit is configured to determine a cross-correlation function, preferably a cumulative maximum cross-correlation function, for a set of detection signals along the axial dimension, and to determine the axial displacement of the course of the first detection signals relative to the course of the second detection signals based on the cross-correlation function. This embodiment is based on the approach to observe disruptions of the ultrasound wave front due to axial or vertical displacement of the object, e.g. a skin region, being imaged during data acquisitions, wherein the cross-correlation function which depends on the axial or vertical displacement between groups of A-lines captures and quantifies the magnitude or height of the disruptions in the sinogram due to axial or vertical motion of the object, e.g. skin. The magnitudes or heights are used to correct for sample movement in the axial dimension during data acquisition. In general, cross-correlation is a measure of similarity of two series as a function of the displacement of one relative to the other. In present case, the cross-correlation function relates to the cross-correlation between groups of A-lines, i.e. groups or sets of detection signals along the axial dimension, and, therefore, maps the disruptions and/or quantifies the axial or vertical displacement(s) of the sets of A-lines relative to each other. Once the vertical displacement is known, the images can be reconstructed accurately based on accordingly corrected detection signals. Advantageously, present embodiment of the motion correction algorithm does not require segmentation of any anatomical area or component of the object, e.g. the skin, and is fully automatic. In this way, a significant reduction of motion artifacts and improvement of resolution (up to 5-fold better than without correction) in the final images is achieved in a particularly simple and reliable way.

Preferably, the processing unit is configured to determine the axial displacement of the course of the first detection signals relative to the course of the second detection signals further based on a smoothed cross-correlation function. Preferably, the processing unit is configured to determine the smoothed cross-correlation function by smoothing the cross-correlation function, e.g. by applying a moving average filter and/or a median filter to the cross-correlation function. Preferably, the processing unit is configured to determine the axial displacement of the course of the first detection signals relative to the course of the second detection signals based on a difference between the cross-correlation function and the smoothed cross-correlation function. Determining the axial displacement based on both the cross-correlation function and the smoothed cross-correlation function is a reliable and simple way to determine the axial displacement, in particular because it is based just on the detection signals and does not require further information input regarding absorption and/or structural properties of the object.

Further advantages, features and examples of the present invention will be apparent from the following description of following figures:

Fig. 1    principle of a motion correction algorithm for RSOM;

Fig. 2    an example of an implementation of the motion correction algorithm in RSOM100;

Fig. 3    an example of an implementation of the motion correction algorithm in clinical RSOM55;

Fig. 4    an example of an implementation of the motion correction algorithm in MSOM;

Fig. 5    an example of an implementation of the motion correction algorithm using a cross-correlation function; and

Fig. 6    a side view of an example of an RSOM system.

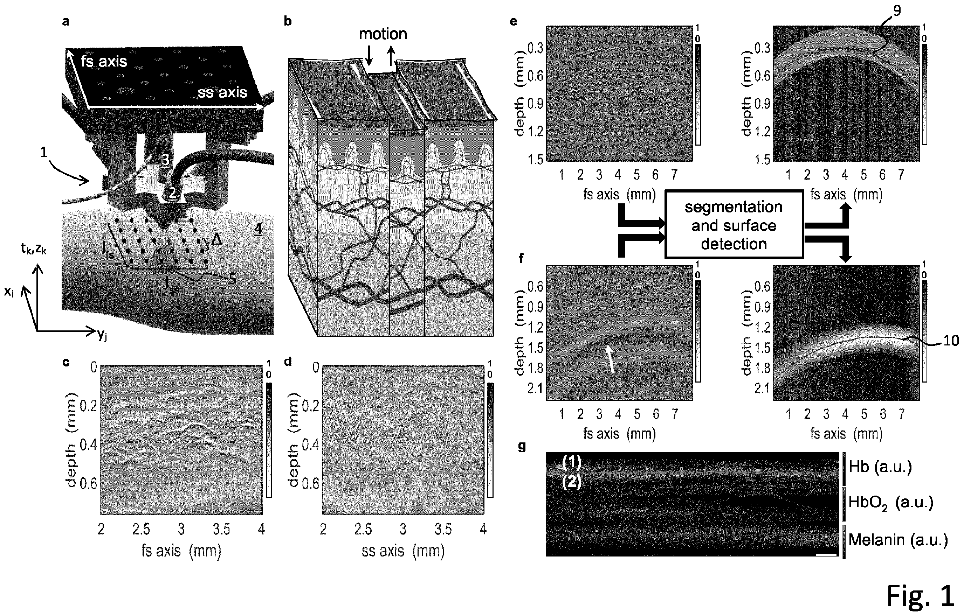

In short, figure 1 (a) to (g) shows examples to illustrate the principle of a motion correction algorithm for RSOM as follows (scale bar: 250 $\mu$m):

(a) Schematic of the detector-illumination unit 1, also referred to as "scan head", which is raster-scanned over a region of interest (ROI) on or in biological tissue 4, in particular skin tissue, at a step size of $\Delta$ to give final dimensions $l_{fs}$ x $l_{ss}$. The detector-illumination unit 1 comprises an illumination unit 2 configured to irradiate the region of interest with electromagnetic radiation, and a detector 3 featuring a cone-shaped sensitivity field 5 and being configured to detect acoustic waves generated in the region of interest at a plurality of locations along an axial dimension z and along two lateral dimensions x, y, which are perpendicular to the axial dimension z, in response to irradiating the region of interest with the electromagnetic radiation and to generate a plurality of according detection signals. For example, the illumination unit 2 comprises an optical fiber and a laser (not shown) emitting electromagnetic radiation which is coupled into the optical fiber and guided to the scan head 1, where it exits the fiber and impinges on the region of interest. In present example, the x dimension is also referred to as fast scan axis or "fs axis", and the y dimension is also referred to as slow scan axis or "ss axis".

(b) Schematic illustrating how movement (black arrows) during RSOM scanning causes discontinuities in the surface of the epidermal layer of skin tissue as well as disruption of vessels.

(c) B-scan of healthy human skin along the fs axis, showing no discontinuities between subsequent positions.

(d) B-scan of the sample in panel c along the ss axis, showing large motion-induced artifacts.

(e) B-scan of healthy human palm (left panel) after segmentation and surface detection were applied (right panel). As indicated by curve 9, the *stratum corneum* is separated clearly from the dermal papillae.

(f) B-scan of healthy lower arm (left panel) with segmentation and surface detection applied (right panel). Segmentation was performed using only the low-frequency optoacoustic signal 600 $\mu$m below the skin surface (arrow). The curve 10 indicates the maximum value of the low-frequency band.

(g) Multispectral vertical MAP of healthy lower arm reconstructed without bandpass filtering. The low-frequency signal (2) shows the spectral signature of melanin and follows the shape of the epidermal layer (1).

Following abbreviations are used: fs: fast-scanning; Hb: deoxyhemoglobin; $HbO_2$: oxyhemoglobin; ss: slow-scanning.

More specifically, RSOM involves raster-scanning a focused ultrasound detector 3 over the ROI and recording the ultrasound waves generated in tissue in response to pulsed laser illumination 2 (Figure 1a). Preferably, the collected data are tomographically reconstructed into an image using three-dimensional beam forming. Typically, the ultrasound detector 3 is scanned along a fast-scanning axis (fs-axis or x-axis) and a slow-scanning axis (ss-axis or y-axis) (Figure 1a). One-dimensional time signals (making up a so-called "A-scan") are recorded at each lateral scan point along the x- and y-axis, collecting ultrasound signal generated within the cone-shaped sensitivity field 5 of the focused detector 3. One-dimensional scanning of the detector 3 along the fs-axis or x-axis generates a two-dimensional sinogram called "B-scan", and scanning along the ss-axis or y-axis generates a stack of B-scans making up a three-dimensional sinogram.

Assuming an acquisition step size of $\Delta$ and an ROI spanning $l_{fs}$ along the fs-axis and a pulse repetition rate (PRR), acquisition speed along the fs-axis is $v_{fs} = PRR \Delta$ and along the ss-axis is $v_{ss} = v_{fs} \Delta/l_{fs}$. Since the step size is much smaller than the dimensions of the ROI, $v_{fs} >> v_{ss}$. Clinical RSOM typically involves $v_{fs} = 15$ mm/s and $v_{ss} = 0.045$ mm/s. These speeds are such that patient motion can alter the axial position of the transducer relative to the ROI between successive B-scans (Figure 1b). Consequently, two-dimensional sinograms along the ss-axis appear discontinuous and rippled (Figure 1d), whereas cross-sections along the fs-axis appear continuous and smooth (Figure 1c).

A preferred first embodiment of a correction algorithm is based on the observation that the ultrasound wave front generated by the melanin-containing layers of the skin (primarily the basal layer of the epidermis and the stratum corneum) show vertical disruptions in the three-dimensional sinogram. According to the Huygens-Fresnel principle, an ultrasound wave emitted from a continuous surface will generate a secondary wave front with a continuous footprint in the three-dimensional sinogram. Movement during RSOM measurement distorts this footprint and generates discontinuities in the three-dimensional sinogram (Figure 1d). Present motion correction algorithm detects disruptions of the stratum corneum/stratum basale (hereafter "skin surface") and corrects for this motion by creating an artificial continuous surface. The primary assumption in this algorithm is that the skin surface is continuous on a mesoscopic scale, i.e. on the order of several micrometers.

Preferably, the motion correction algorithm proceeds in two steps. First, an algorithm is applied to detect the discontinuous skin surface in the three-dimensional sinogram. Second, an artificial, smooth, continuous skin surface is generated based on the detected surface, and the difference between the two surfaces is taken to indicate the vertical movement of the skin with respect to the detector surface. Based on this vertical movement, the focal point of the detector is adjusted during image reconstruction.

Preferably, the first step of the algorithm involves detecting the skin surface in the three-dimensional sinogram. To this end, different approaches may be applied, preferably depending on the tissue being examined.

For example, when the ROI lies in an area of hairless skin (e.g. palm of the hand), the stratum corneum is roughly segmented using a two-dimensional parabolic slab, reflecting the fact that optoacoustic signal from the stratum corneum is displaced by several hundred microns from the signal emitted from underlying vasculature (Figure 1e). Within the segmented slab, the surface is identified based on the maximum value within each A-scan.

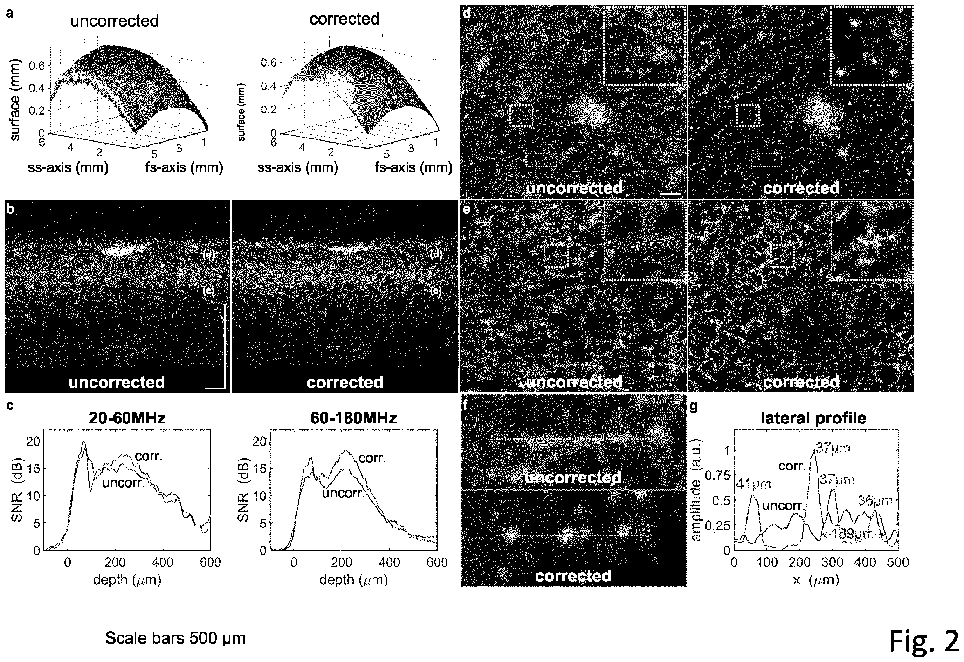

For example, when the ROI lies in an area of hairy skin (e.g. lower arm), another approach is used because the stratum corneum is not sufficiently separated in space from the underlying stratum basale and dermal papillae (Figure 1f). Nonetheless, the basal layer of the epidermis leaves a distinctive footprint in the three-dimensional sinogram: ultrasound signals emitted by the stratum basale induce shear waves within the acoustic lens of the detector, which in turn stimulate lateral modes in the piezo element of the detector, generating a characteristic low-frequency footprint approximately 600 μm below the skin surface (Figure 1g). Usually, this low-frequency band is filtered out before reconstruction, but present algorithm exploits it to generate an image of the skin surface. An exponential bandpass filter from 1 to 3 MHz is applied to the raw data (detection signals), which are roughly segmented by time to identify the interval containing the highest peak (Figure 1f). The maximum in the low-frequency band is determined for each A-scan, resulting in a two-dimensional map of the motion-corrupted melanin layer (Figure 2a).

Preferably, the second step in the algorithm is the same regardless of whether the skin ROI is hairy or hairless. A moving average filter is applied to the disrupted surface, correcting the entire three-dimensional sinogram and creating an artificially smoothed surface (Figure 2b). Preferably, the moving average filter spans 200 pixels along the ss-axis and 10 pixels along the fs-axis. A two-dimensional map between the motion-corrupted and artificially smoothed surfaces is calculated to provide the correction for detector z-position during reconstruction.

Preferably, the motion correction algorithm is applied to in vivo data collected with various RSOM set-ups. Preferable, the RSOM set-ups comprise a spherically focused ultrasound detector with acoustic lens.

For example, a high-frequency RSOM set-up (hereafter "RSOM100") is used to image small vessels in a benign nevus on the lower arm of a healthy man with skin type II. Preferably, the detector has a detection bandwidth of 20-180 MHz, an active aperture of 1.5 mm and an f-number of 1.1. To ensure fast, high-resolution scans, a single-wavelength laser emitting pulses of 532-nm light lasting <1 ns at repetition rates up to 2 kHz is used.

A clinical RSOM set-up (hereafter "RSOM55"), in which the scan head is attached to a portable clinical imaging platform, is preferably used to image healthy skin as well as a, e.g., psoriasis plaque on the skin. The RSOM55 detector has a preferred detection bandwidth of 14-126 MHz, an active aperture of 3 mm, and an f-number of 1. The laser type is preferably the same as in the RSOM100 set-up.

During image reconstruction of data collected with the RSOM100 or RSOM55 set-ups, the reconstruction bandwidth is preferably split up into low- and high-frequency sub-bands, which were 20 60 and 60 180 MHz for RSOM100 or 14-42 and 42-126 MHz for RSOM55. The two bands were equalized, colored green (high-frequency) or red (low-frequency), and overlaid in final images. Reconstruction using frequency sub-bands improves visualization of higher frequency signals emitted by smaller structures in the tissue.

Another preferred set-up, multispectral RSOM (hereafter "MSOM"), may be used, e.g., to measure blood oxygenation in an extremity of an individual, e.g. in the lower arm of a healthy man with skin type III. The detector is preferably the same as in the RSOM55 set-up but with a preferred detection bandwidth of 14-126 MHz. Tissue is preferably excited using a tunable-wavelength OPO laser emitting 6-ns pulses in the spectral range from 420 to 2300 nm at a pulse repetition rate of 100 Hz.

Uncorrected and corrected reconstructions obtained using the RSOM100 and RSOM55 set-ups may be compared in terms of their contrast-to-noise ratio (CNR) as a function of depth. CNR was defined as

$$CNR(z_k) = 20 * \log_{10}[R_{max}(z_k) / noise_{max}], \tag{1}$$

where $R_{max}(z_k) = \max_{i,j} R(x_i, y_j, z_k)$ is the maximal amplitude within the image plane at depth $z_k$, and $noise_{max}$ is the background signal based on the maximal amplitude of the image above the skin surface. The term $R(x_i, y_j, z_k)$ represents the three-dimensional reconstruction, where $i = 1,2,3, ... , N_x$; $j = 1,2,3, ... , N_y$; and $k = 1,2,3, ... , N_z$, and $N_x$, $N_y$, and $N_z$ are the numbers of voxels along the x-, y-, and z-axes. The plot of $CNR(z_k)$ as a function of depth should show smaller peaks for motion-corrupted data than for data appropriately motion-corrected.

Further, uncorrected and corrected RSOM100 images may be also compared in terms of resolution calculated from the full width at half maximum (FWHM) of optoacoustic peaks along line profiles corresponding to single capillary loops in the skin. The top part of capillary loops appears as a circular absorber in RSOM images.

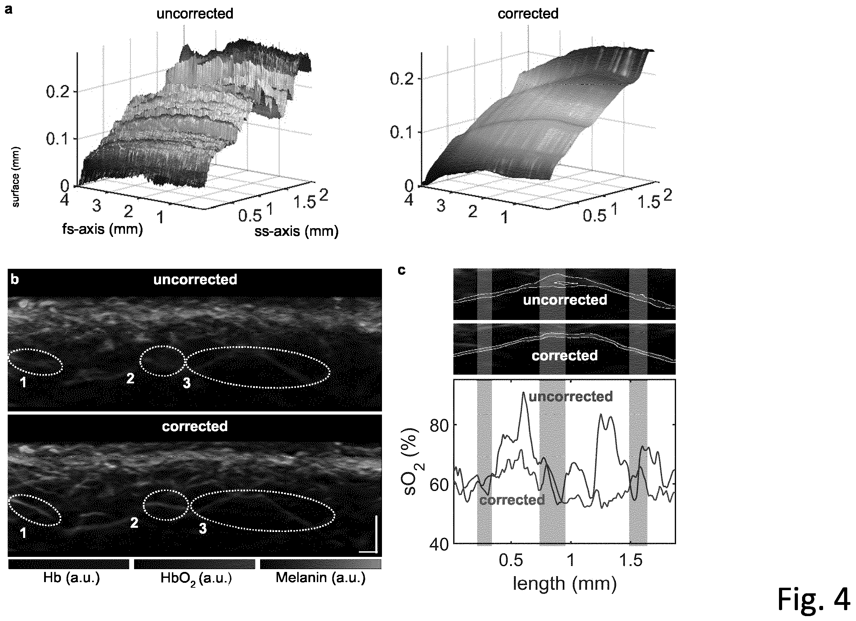

Uncorrected and corrected MSOM images may be compared in terms of blood vessel visualization and blood oxygenation measurement. For blood oxygenation analysis, the same vessel was segmented in motion-corrupted and motion-corrected three-dimensional reconstructions, and blood oxygenation was measured along the vessel (Figure 4). The segmented vessel was oriented primarily along the y-axis. Blood oxygenation at position $y_{j'}$ was averaged across the diameter of the vessel using the equation

$$sO_2\left(y_{j'}\right) = \frac{\sum_{i',k'}\left(Seg(x_{i'},y_{j'},z_{k'})*sO_2(x_{i'},y_{j'},z_{k'})\right)}{\sum_{i',k'}\left(Seg(x_{i'},y_{j'},z_{k'})\right)},\qquad(2)$$

where $sO_2(x_{j'},y_{j'},z_{k'})$ is the voxel-to-voxel ratio of oxygenated hemoglobin to total hemoglobin (oxyhemoglobin plus deoxyhemoglobin). The vessel was segmented by thresholding the reconstructed images at 20% of the maximal value:

$$Seg\left(x_{i'},y_{j'},z_{k'}\right) = H\left(R\left(x_{i'},y_{j'},z_{k'}\right) - 0.2*\max_{i',j',k'}\left[R\left(x_{i'},y_{j'},z_{k'}\right)\right]\right),\qquad(3)$$

where H(x) is the Heaviside step function and $R(x_{j'},y_{j'},z_{k'})$ describes a cuboid in the reconstructed volume that contains the vessel of interest. $Seg(x)$ = 1 if voxel amplitude is greater than 20% of the maximum amplitude within the cuboid; otherwise, $Seg(x)$ = 0. Blood oxygenation level $sO_2(y_{j'})$ along the length of blood vessels was compared before and after motion correction. Motion during MSOM data acquisition should give rise to abrupt or large fluctuations in $sO_2$ that do not overlap with vessel bifurcations.

Figure 2 (a) to (g) shows examples to illustrate an implementation of the motion correction algorithm in RSOM100 as follows (scale bar: 500 $\mu$m):

(a) Skin surface detected in the raw data before and after motion correction.

(b) Vertical MAPs of a region of interest on the lower arm surrounding a benign nevus, before and after motion correction. The labels "(d)" and "(e)" mark the location of the epidermal-dermal junction and the dermal layer shown as lateral MAPs in panels d and e respectively.

(c) Mean CNR in the cross-sectional MAPs in panel b as a function of depth before and after motion correction. Reconstructions were based on the frequency bands 20-60 MHz (left) or 60-180 MHz (right).

(d) Lateral MAP of the epidermal-dermal junction before and after motion correction. The insets are zoomed-in views of the areas enclosed in white dashed boxes, showing single capillary loops after motion correction. Solid-line boxes enclose areas shown as zoomed-in views in panel f.

(e) Lateral MAP of an extensive vascular network in the dermal layer, showing well-resolved microvessels after motion correction.

(f) Zoomed-in views of the regions boxed with solid lines in panel d. White dashed lines trace the lateral profile in panel g.

(g) Lateral profile through four distinct capillary loops. Peak FWHM values are shown.

More specifically, figure 2 illustrates the performance of the motion correction algorithm when imaging a benign nevus on the lower arm of a healthy volunteer using the RSOM100 set-up. The algorithm identified a rippled surface in the raw data, indicating micromotion in the z-direction on the order of several micrometers between successive B-scans (Figure 2a). The skin surface appeared disrupted only along the ss-axis, not along the fs-axis. The motion-corrected skin surface was continuous and smooth, with no offset between neighboring B-scans. Figure 2b displays the vertical maximum amplitude projections (MAPs) of motion-corrupted and motion-corrected imaging volumes. Blurring was visible in the uncorrected image, especially in the high-frequency (green) reconstruction. Figure 2c plots mean CNR with respect to depth based on the vertical MAPs. Motion correction led to higher CNR in the stratum corneum as well as superficial horizontal plexus; the CNR increase over uncorrected data was greater at high frequencies (up to 5 dB) than at low ones (up to 2.5 dB). In addition to this quantitative increase in CNR, motion correction led to significant qualitative improvement in the resolution of the epidermal-dermal junction and the dermis in lateral MAPs (Figure 2d-e). The increase in resolution was particularly apparent at the epidermal-dermal junction (Figure 2d): the uncorrected MAP showed an unstructured pattern, while the motion-corrected MAP showed a structured pattern of capillary loops. The zoomed-in region (500 $\mu$m x 500 $\mu$m) in Figure 2d revealed no individual capillary loops before motion correction, whereas more than a dozen loops were visible after correction. Similarly, microvessels in the papillary dermis were resolved only after motion correction. Inset images in Figure 2e suggest that micromotion affected small vessels much more than large ones.

To quantify the improvement in resolution associated with the motion correction algorithm, line profiles were traced through neighboring capillary loops and analyzed (Figure 2f-g). While the uncorrected image showed several neighboring capillaries as a lateral tube without clear peaks in the line profile, the corrected image showed four distinctive capillary loops, each appearing as a resolved peak with FWHM values of 36-41 $\mu$m. Peaks 3 and 4 combined to form a plateau with FWHM of 189 $\mu$m. A walk-through of the motion-corrected three-dimensional volume of the ROI illustrates good resolution of vascular networks at various depths (Supplementary Movie S1).

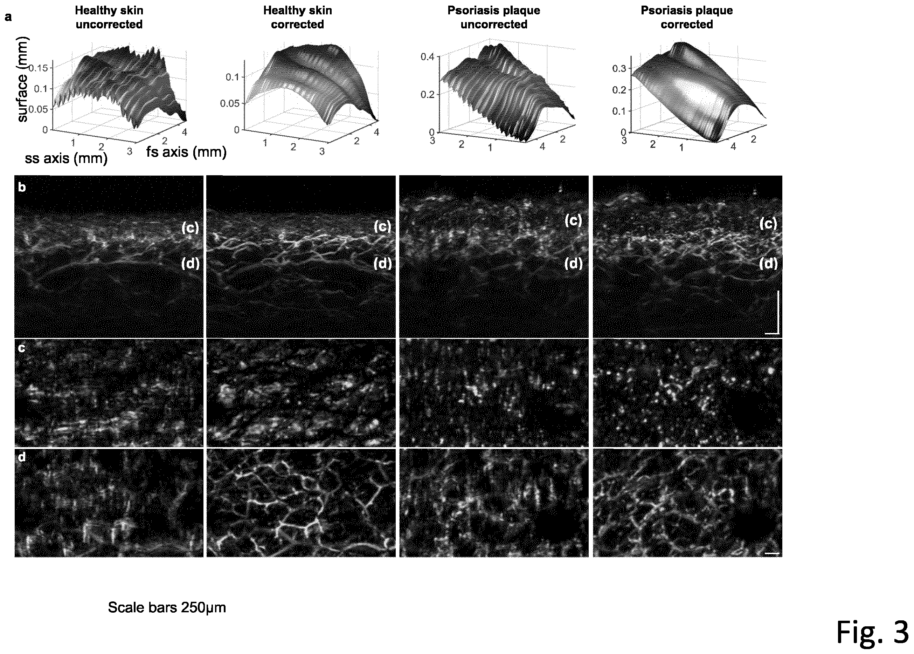

Figure 3 (a) to (d) shows examples of an implementation of the motion correction algorithm in clinical RSOM55 as follows (scale bar: 250 $\mu$m):

(a) Skin surface detected in the raw data before and after motion correction. Periodic disruptions due to breathing are visible in the uncorrected data.

(b) Vertical MAPs before and after motion correction. Psoriatic skin shows typical thickening of epidermis, broadening of capillary loops, and increased blood volume fraction. The labels "(c)" and "(d)" mark the location of the epidermal-dermal junction and the dermal layer shown as lateral MAPs in panels c and d respectively.

(c) Lateral MAP of the epidermal-dermal junction. Broadening of capillary loops in psoriatic skin is visible after motion correction. Capillaries in healthy skin are too small to be captured by RSOM55.

(d) Lateral MAP of an extensive vascular network in the dermal layer, showing well-resolved microvessels only after motion correction.

More specifically, figure 3a reveals sinusoidal modulation of healthy and psoriatic skin surfaces at approximately 15 cycles per minute (17-18 cycles per measurement), corresponding to the patient's breathing. This sinusoidal movement was eliminated by the motion correction algorithm, allowing the visualization of inflammation biomarkers typical of psoriatic skin, including thickening of the epidermis, broadening of capillary loops within the dermal papillae, and increased blood volume fraction in dermal vasculature (Figure 3b to d). Motion correction significantly improved image quality: capillary loops in the psoriasis plaque appeared blurred before motion correction and well resolved afterwards (Figure 3c). Perhaps the greatest improvement in image quality was observed in the microvasculature of the superficial horizontal plexus (Figure 3d). Images of both healthy and psoriatic skin revealed a structured network of smaller vessels and larger ones running through the dermis. Smaller vessels were completely blurred before motion correction but well resolved afterwards.

Figure 4 (a) to (c) shows examples of an implementation of the motion correction algorithm in MSOM as follows (scale bar: 250 $\mu$m):

(a) Skin surface of healthy lower arm area before and after motion correction. Strong motion artifacts are visible even along the fs-axis because of the long measurement time necessary for multispectral acquisition. Nevertheless, the algorithm is able to generate a smooth, continuous surface.

(b) Vertical MAP of unmixed chromophores in the lower arm. Individual vessels enclosed by ellipses 1 and 2 are resolved only after motion correction. The vessel enclosed by ellipse 3 is further analyzed in panel c.

(c) Segmentation of the vessel within ellipse 3 in panel b and outlined in white (top and middle panels), followed by measurement of blood oxygenation (oxygen saturation, sO$_2$) across the diameter of the blood vessel within the segmented area (lower panel). The oxygenation level is plotted from left to right along the vessel. Gray bands indicate regions of vessel bifurcation.

More specifically, by measuring three-dimensional absorption maps at multiple wavelengths, MSOM provides functional information based on the spectral signature of intrinsic chromophores. Figure 4 shows the impact of motion on the visualization of vasculature as well as the quantification of blood oxygenation in the lower arm of a healthy volunteer. Figure 4a shows that without motion correction, the skin surface appeared strongly rippled, even along the fs-axis, reflecting the prolonged measurement time needed for MSOM data acquisition. The motion-corrected surface was smooth and continuous along both the fs- and ss-axes. Figure 4b highlights two areas (ellipses 1 and 2) where single blood vessels appeared as a cloud without clear boundaries prior to motion correction but as well-resolved vessels afterwards. Another vessel (ellipse 3) appeared as a double structure before motion correction, and the "shadow vessel" disappeared after correction (Figure 4b-c).

In addition to affecting blood vessel visualization in MSOM, motion also interfered with measurement of blood oxygenation. The vessel within ellipse "3" in Figure 4b was segmented and a mean blood oxygenation was measured along its length (Figure 4c). Blood oxygenation level in the uncorrected image fluctuated sharply between 55% and 90%, and the fluctuations did not correlate with vessel bifurcations (gray bands). Blood oxygenation level in the motion-corrected image showed smaller, less abrupt variations, ranging from 55% to 70%. In addition, regions of vessel bifurcation were associated with changes in blood oxygenation.

The algorithm described above works by identifying the footprint of the skin surface in three-dimensional sinograms, which is facilitated by the strongly absorbing stratum basale. In the case of humans or animals lacking an absorbing layer close to the skin surface, such a layer can easily be created by dissolving ink in the acoustic coupling agent, by slightly coloring the skin surface with a marker, or by introducing absorbing molecules in the coupling membrane. This is analogous to marking the skin surface with a fiducial marker in optical coherence tomography. Thus, the motion correction algorithm described herein is applicable to a wide range or preclinical and clinical RSOM studies.

This motion correction algorithm helps provide the accuracy and reliability needed for realizing the full clinical potential of RSOM. The RSOM technique, already optimized in terms of frequency and excitation energy, has shown the ability to measure angiogenesis in preclinical models of melanoma tumors, and phenotypic biomarkers of inflammation in human skin. The multispectral capability of MSOM can provide readings of dermal blood oxygenation level and melanin content. Therefore, the algorithm described herein allows for a wide range of applications in the field of RSOM-based dermatology.

Alternatively or additionally to the first embodiment of a motion correction algorithm described above, a preferred second embodiment of a motion correction algorithm quantifies the height of the disruption (discontinuities) of the sinogram in the ss-axis. To that end, a cumulative maximum cross-correlation function is defined as follows:

$$ccorr(x_i, y_N) = \sum_{j=1}^{N-1} ccorr(x_i, y_j) + \arg\max_d \left( \sum_{k,i} b(t_k, x_i^l, y_N) b(t_{k-d}, x_i^l, y_{N+1}) \right) \quad (4)$$

Where $x_i$ and $y_j$ are the positions of the acquisition grid in the fast scan (fs) and slow scan (ss) axis, respectively. Each pair (i,j) defines the position of an A-line defined as $f(t_k, x_i, y_j)$. A section of a B-plane containing $l$ A-lines is ( $t_k, x_i^l, y_j$ ), where the A-lines range from $f(t_k, x_{i-l/2}, y_j)$ to $f(t_k, x_{i+l/2}, y_j)$. Under no motion conditions, the function $ccorr(x_i, y_N)$ is of C1 class, that is, it exhibits a smooth course.

Preferably, in the second embodiment the algorithm works as follows: First, the cumulative maximum cross-correlation function is calculated. Second, a new, smooth, cumulative cross-correlation function is calculated, and the difference between both functions is taken to indicate the axial or vertical movement of the object, e.g. the skin, with respect to the ultrasound detector surface. Based on this vertical movement, the focal point of the detector is adjusted during image reconstruction.

In the first step of the algorithm, the cumulative maximum correlation distance function is calculated, which is a fully automatic process. The only parameter that has to be selected beforehand is $l$. As a result, a 2-dimensional function is obtained which is smooth in the absence of motion during data acquisition. In case of motion during data acquisition, however, the 2-dimensional function appears disrupted. Preferably, a moving average filter is applied to the disrupted surface, i.e. function. Preferably, a two-dimensional map between the motion-corrupted function and artificially smoothed function is calculated to provide the correction for detector z-position during reconstruction.

Similarly to the first embodiment of a motion correction algorithm described above, the second embodiment of a motion correction algorithm allows for efficient and reliable motion correction of optoacoustic signals acquired with the RSOM systems described above, in particular signals acquired with RSOM55 from a healthy skin. Advantageously, with this algorithm the resolution of single capillary loops is up to 5-fold better than before or without correction. Likewise, for healthy and psoriatic skin imaging with the clinical RSOM system (14 to 126 MHz) a correction of breathing motion is achieved, which allows for reliable imaging in a clinical context.

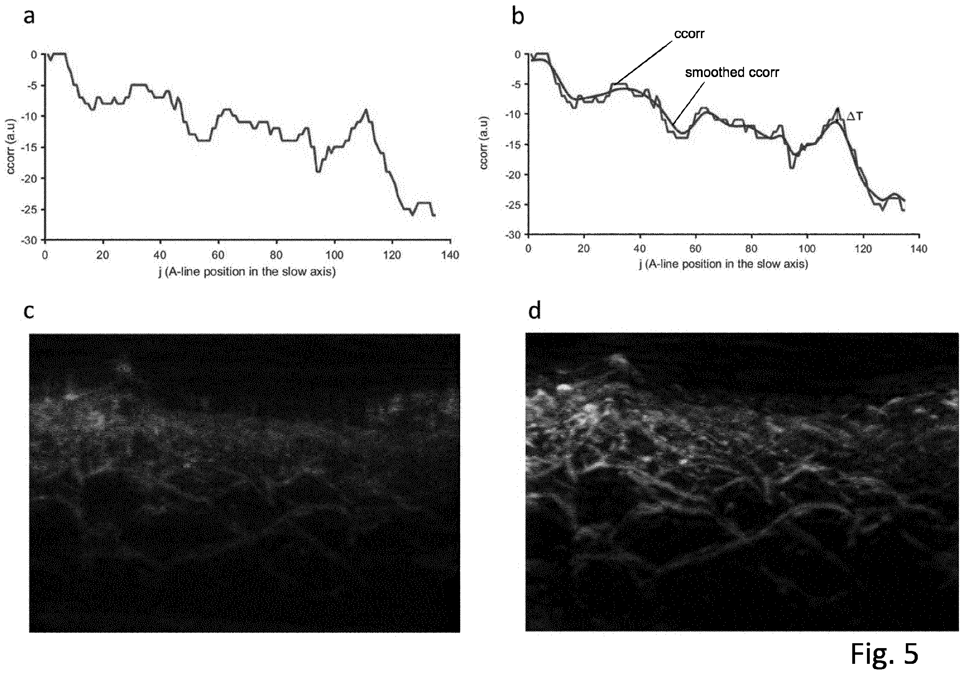

This is exemplarily illustrated in Figure 5 a), which shows a cross-correlation function (ccorr) for a given A-line defined by the $x_i$ position of the grid. It can be clearly seen that the cross-correlation function is not smooth. Figure 5 b) shows a smoothed cross-correlation (smoothed ccorr) function, from which the distances ($\Delta T$) to the non-smooth function (ccorr) are calculated. Figure 5 c) shows a reconstructed image without motion correction, whereas Figure 5 d) shows a reconstructed image after motion correction using the cross-correlation function.

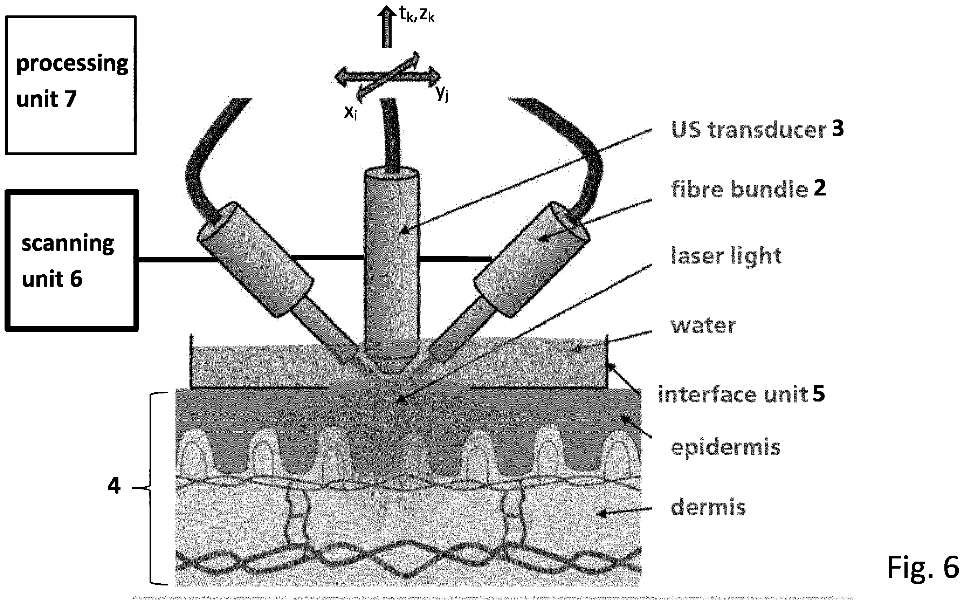

Figure 6 shows a schematic side view of an example of an RSOM system comprising an illumination unit 2, e.g. two fiber bundles, by which transient laser light is guided and irradiated to the object 4, e.g. skin tissue. A detection unit 3 comprises an ultrasound (US) transducer which detects acoustic waves which are generated in the object 4 in response to irradiation with the laser light. In order to ensure good acoustic and optical coupling, an interface unit 5, also referred to as "coupling unit", is provided. In present example, the coupling unit comprises a membrane, which is in contact with the skin surface, and a frame on which the membrane is attached. The frame is filled with water as a coupling medium.

The system further comprises a scanning unit 6 which is configured to move the detection unit 3 and/or illumination unit 2, on the one hand, and the object 4, on the other hand, along at least one lateral dimension x and/or y relative to each other. Preferably, the scanning unit 6 is configured to move both the detection unit 3, e.g. the transducer, and the illumination unit 2, e.g. the ends of fiber bundles, along two lateral dimensions x and y.

The scanning unit 6 is further configured to control the detection unit 3 to detect acoustic waves generated in the object 4 at a plurality of different locations $x_i$ and/or $y_j$ along the at least one lateral dimension in response to irradiating the object 4 with electromagnetic radiation and to generate a plurality of according detection signals, so-called A-scans, at time points $t_k$ at positions $z_k$ along an axial dimensions at the plurality of positions $x_i$ and/or $y_j$ along the at least one lateral direction.

In this way, a three-dimensional set $x_i$, $y_j$, $t_k$ (or $z_k$) of detection signals is obtained, which contains information on the object 4, e.g. light-absorbing properties of components of the skin tissue, along the at least one lateral dimension x and/or y, and along the axial dimension z, which is perpendicular to the at least one lateral dimension.

A processing unit 7 is configured to correct the detection signals for an axial or vertical displacement of the object 4 in the z dimension during the detection of the acoustic waves by applying a motion correction algorithm described herein.

The processing unit 7 is further configured to reconstruct at least one image of the object 4 based on the motion-corrected detection signals.

**Claims**

1. A system for optoacoustic imaging comprising

   an irradiation unit (2) configured to irradiate an object (4) comprising biological tissue with electromagnetic radiation,
   a detection unit (3) configured to detect acoustic waves generated in the object (4) at a plurality of locations along an axial dimension (t) and along at least one lateral dimension (x, y), which is perpendicular to the axial dimension (t), in response to irradiating the object with electromagnetic radiation and to generate a plurality of according detection signals (I(x, y, t)), and
   a processing unit (7) configured

   - to determine an axial displacement ($\Delta t$) of the course of one or more first detection signals along the axial dimension (t) relative to the course of one or more second detection signals along the axial dimension (t),
   - to correct the detection signals by reducing and/or eliminating the axial displacement ($\Delta t$), and
   - to reconstruct at least one two- or three-dimensional image of the object based on the corrected detection signals.

2. The system according to claim 1, the processing unit being configured

   - to detect a structure contained in the detection signals, the detected structure corresponding to a representation of a component of the object in the detection signals, and
   - to determine the axial displacement ($\Delta t$) of the course of the first detection signals relative to the course of the second detection signals based on an axial offset of a section of the detected structure relative to an expected structure, which corresponds to the component of the object, along the axial dimension (t).

3. The system according to claim 2, the detected structure corresponding to a representation of an endogenous component of the tissue.

4. The system according to claim 3, the endogenous component of the tissue being at least one of the following: at least one melanin-containing layer of skin tissue, the basal layer (stratum basale) of the epidermis of skin tissue, the cornified layer (stratum corneum) of the epidermis of skin tissue, a layer of blood vessels of the tissue.

5. The system according to any of the claims 2 to 4, the detected structure corresponding to a representation of an exogenous layer configured to absorb at least a part of the electromagnetic radiation.

6. The system according to claim 5, the exogenous layer corresponding to a coupling element arranged between the tissue and the detection unit and configured to acoustically couple the detection unit with the tissue and/or a medium applied to the surface of the tissue, in particular to the skin.

7. The system according to any of the claims 2 to 6, the processing unit being configured to enhance and/or detect the structure contained in the detection signals by applying a bandpass filter to the detection signals and/or a segmentation of information contained in the detection signals.

8. The system according to any of the claims 2 to 7, the processing unit being configured to determine the section of the detected structure, which is offset relative to the expected structure along the axial dimension (t), based on a, preferably rough, segmentation of information contained in the detection signals along the axial dimension (t) and along at least one lateral dimension (x, y).

9. The system according to any of the claims 2 to 8, the processing unit being configured to determine a map of the detected structure along the at least one lateral dimension (x, y) and to determine a map of the expected structure along the at least one lateral dimension (x, y) based on the map of the detected structure.

10. The system according to claim 9, the processing unit being configured to determine the map of the expected structure by smoothing the map of the detected structure.

11. The system according to claim 10, the processing unit being configured to smooth the map of the detected structure by applying a median filter and/or a moving average filter to the map of the detected structure along at least one lateral dimension (x, y).

12. The system according to any of the claims 9 to 11, the processing unit being configured to determine the axial offset of the section of the detected structure relative to the expected structure along the axial dimension (t) based on the map of the detected structure and the map of the expected structure.

13. The system according to any of the preceding claims, the processing unit being configured

- to determine a cross-correlation function ($ccorr(x_i, y_j)$), preferably a cumulative maximum cross-correlation function, for detection signals along the axial dimension (t), wherein the cross-correlation function ($ccorr(x_i, y_j)$) relates to the cross-correlation between the detection signals along the axial dimension (t), and
- to determine the axial displacement ($\Delta t$) of the course of the first detection signals relative to the course of the second detection signals based on the cross-correlation function ($ccorr(x_i, y_j)$).

14. The system according to claim 13, the processing unit being configured to determine the axial displacement ($\Delta t$) of the course of the first detection signals relative to the course of the second detection signals further based on a smoothed cross-correlation function.

15. The system according to claim 14, the processing unit being configured to determine the axial displacement ($\Delta t$) of the course of the first detection signals relative to the course of the second detection signals based on a difference between the cross-correlation function ($ccorr(x_i, y_j)$) and the smoothed cross-correlation function.

16. The system according to claim 15, the processing unit being configured to determine the smoothed cross-correlation function by smoothing the cross-correlation function ($ccorr(x_i, y_j)$).

17. The system according to claim 16, the processing unit being configured to determine the smoothed cross-correlation function by applying a moving average filter and/or a median filter to the cross-correlation function ($ccorr(x_i, y_j)$).

18. A method for optoacoustic imaging data processing implemented on a processing unit, the method comprising the following steps:

- retrieving a plurality of detection signals ($I(x, y, t)$) representing acoustic waves generated in an object (4) at a plurality of locations along an axial dimension (t) and along at least one lateral dimension (x, y), which is perpendicular to the axial dimension (t), in response to irradiating the object (4) with electromagnetic radiation,
- determining an axial displacement ($\Delta t$) of the course of one or more first detection signals along the axial dimension (t) relative to the course of one or more second detection signals along the axial dimension (t),
- correcting the detection signals by reducing and/or eliminating the axial displacement ($\Delta t$), and
- reconstructing at least one two- or three-dimensional image of the object based on the corrected detection signals.

**Patentansprüche**

1. System zur optoakustischen Bildgebung, aufweisend

eine Bestrahlungseinheit (2), die so konfiguriert ist, dass sie ein Objekt (4), das biologisches Gewebe aufweist, mit elektromagnetischer Strahlung bestrahlt,
eine Detektionseinheit (3), die so konfiguriert ist, dass sie akustische Wellen, die in dem Objekt (4) an einer Vielzahl von Orten entlang einer axialen Dimension (t) und entlang mindestens einer lateralen Dimension (x, y), die senkrecht zu der axialen Dimension (t) ist, als Reaktion auf die Bestrahlung des Objekts mit elektromagnetischer Strahlung erzeugt werden, detektiert und eine Vielzahl von entsprechenden Detektionssignalen (I(x, y, t)) erzeugt, und
eine Verarbeitungseinheit (7), die so konfiguriert ist, dass sie

   - eine axiale Verschiebung ($\Delta t$) des Verlaufs eines oder mehrerer erster Detektionssignale entlang der axialen Dimension (t) relativ zum Verlauf eines oder mehrerer zweiter Detektionssignale entlang der axialen Dimension (t) bestimmt,
   - die Detektionssignale korrigiert, indem sie die axiale Verschiebung ($\Delta t$) reduziert und/oder eliminiert, und
   - mindestens ein zwei- oder dreidimensionales Bild des Objekts auf der Grundlage der korrigierten Detektionssignale rekonstruiert.

2. System nach Anspruch 1, wobei die Verarbeitungseinheit konfiguriert ist,

   - eine in den Detektionssignalen enthaltene Struktur zu detektieren, wobei die detektierte Struktur einer Darstellung einer Komponente des Objekts in den Detektionssignalen entspricht, und
   - die axiale Verschiebung ($\Delta t$) des Verlaufs der ersten Detektionssignale relativ zum Verlauf der zweiten Detektionssignale basierend auf einem axialen Versatz eines Abschnitts der detektierten Struktur relativ zu einer erwarteten Struktur, die der Komponente des Objekts entspricht, entlang der axialen Abmessung (t) zu bestimmen.

3. System nach Anspruch 2, wobei die erfasste Struktur einer Darstellung einer endogenen Komponente des Gewebes entspricht.

4. System nach Anspruch 3, wobei die endogene Komponente des Gewebes mindestens eine der folgenden ist: mindestens eine melaninhaltige Schicht des Hautgewebes, die Basalschicht (Stratum basale) der Epidermis des Hautgewebes, die verhornte Schicht (Stratum corneum) der Epidermis des Hautgewebes, eine Schicht von Blutgefäßen des Gewebes.

5. System nach einem der Ansprüche 2 bis 4, wobei die erfasste Struktur einer Darstellung einer exogenen Schicht entspricht, die so konfiguriert ist, dass sie zumindest einen Teil der elektromagnetischen Strahlung absorbiert.

6. System nach Anspruch 5, wobei die exogene Schicht einem Kopplungselement entspricht, das zwischen dem Gewebe und der Detektionseinheit angeordnet und so konfiguriert ist, dass es die Detektionseinheit akustisch mit dem Gewebe und/oder einem auf der Oberfläche des Gewebes, insbesondere der Haut, aufgebrachten Medium koppelt.

7. System nach einem der Ansprüche 2 bis 6, wobei die Verarbeitungseinheit so konfiguriert ist, dass sie die in den Erfassungssignalen enthaltene Struktur durch Anwendung eines Bandpassfilters auf die Erfassungssignale und/oder eine Segmentierung der in den Erfassungssignalen enthaltenen Informationen verbessert und/oder erfasst.

8. System nach einem der Ansprüche 2 bis 7, wobei die Verarbeitungseinheit so ausgebildet ist, dass sie auf Basis einer, vorzugsweise groben, Segmentierung von in den Detektionssignalen enthaltenen Informationen entlang der axialen Dimension (t) und entlang mindestens einer lateralen Dimension (x, y) den Ausschnitt der detektierten Struktur bestimmt, der gegenüber der erwarteten Struktur entlang der axialen Dimension (t) versetzt ist.

9. System nach einem der Ansprüche 2 bis 8, wobei die Verarbeitungseinheit so konfiguriert ist, dass sie eine Karte der erfassten Struktur entlang der mindestens einen lateralen Dimension (x, y) bestimmt und eine Karte der erwarteten Struktur entlang der mindestens einen lateralen Dimension (x, y) auf der Grundlage der Karte der erfassten Struktur bestimmt.

10. System nach Anspruch 9, wobei die Verarbeitungseinheit so konfiguriert ist, dass sie die Karte der erwarteten Struktur durch Glätten der Karte der erfassten Struktur bestimmt.

11. System nach Anspruch 10, wobei die Verarbeitungseinheit so konfiguriert ist, dass sie die Karte der erfassten Struktur glättet, indem sie einen Medianfilter und/oder einen Filter mit gleitendem Mittelwert auf die Karte der erfassten Struktur entlang mindestens einer lateralen Dimension (x, y) anwendet.

12. System nach einem der Ansprüche 9 bis 11, wobei die Verarbeitungseinheit so konfiguriert ist, dass sie den axialen Versatz des Abschnitts der detektierten Struktur relativ zu der erwarteten Struktur entlang der axialen Dimension (t) auf der Grundlage der Karte der detektierten Struktur und der Karte der erwarteten Struktur bestimmt.

13. System nach einem der vorhergehenden Ansprüche, wobei die Verarbeitungseinheit konfiguriert ist,

- eine Kreuzkorrelationsfunktion (ccorr($x_i$ , $y_j$ )), vorzugsweise eine kumulative maximale Kreuzkorrelationsfunktion, für Detektionssignale entlang der axialen Dimension (t) zu bestimmen, wobei die Kreuzkorrelationsfunktion (ccorr($x_i$ , $y_j$ )) sich auf die Kreuzkorrelation zwischen den Detektionssignalen entlang der axialen Dimension (t) bezieht, und
- die axiale Verschiebung ($\Delta t$) des Verlaufs der ersten Detektionssignale relativ zum Verlauf der zweiten Detektionssignale auf der Basis der Kreuzkorrelationsfunktion (ccorr($x_i$ , $y_j$ )) zu bestimmen.

14. System nach Anspruch 13, wobei die Verarbeitungseinheit so konfiguriert ist, dass sie die axiale Verschiebung ($\Delta t$) des Verlaufs der ersten Erfassungssignale relativ zum Verlauf der zweiten Erfassungssignale ferner auf der Grundlage einer geglätteten Kreuzkorrelationsfunktion bestimmt.

15. System nach Anspruch 14, wobei die Verarbeitungseinheit so konfiguriert ist, dass sie die axiale Verschiebung ($\Delta t$) des Verlaufs der ersten Erfassungssignale relativ zum Verlauf der zweiten Erfassungssignale basierend auf einer Differenz zwischen der Kreuzkorrelationsfunktion (ccorr($x_i$ , $y_j$ )) und der geglätteten Kreuzkorrelationsfunktion bestimmt.

16. System nach Anspruch 15, wobei die Verarbeitungseinheit so konfiguriert ist, dass sie die geglättete Kreuzkorrelationsfunktion durch Glätten der Kreuzkorrelationsfunktion (ccorr($x_i$ , $y_j$ )) bestimmt.

17. System nach Anspruch 16, wobei die Verarbeitungseinheit so konfiguriert ist, dass sie die geglättete Kreuzkorrelationsfunktion durch Anwendung eines Filters für den gleitenden Durchschnitt und/oder eines Medianfilters auf die Kreuzkorrelationsfunktion (ccorr($x_i$ , $y_j$ )) bestimmt.

18. Verfahren zur optoakustischen Bilddatenverarbeitung, das in einer Verarbeitungseinheit implementiert ist, wobei das Verfahren die folgenden Schritte aufweist:

- Abrufen einer Vielzahl von Detektionssignalen (I(x, y, t)), die akustische Wellen darstellen, die in einem Objekt (4) an einer Vielzahl von Orten entlang einer axialen Dimension (t) und entlang mindestens einer lateralen Dimension (x, y), die senkrecht zur axialen Dimension (t) ist, als Reaktion auf die Bestrahlung des Objekts (4) mit elektromagnetischer Strahlung erzeugt werden,
- Bestimmen einer axialen Verschiebung ($\Delta t$) des Verlaufs eines oder mehrerer erster Detektionssignale entlang der axialen Dimension (t) relativ zum Verlauf eines oder mehrerer zweiter Detektionssignale entlang der axialen Dimension (t),
- Korrigieren der Detektionssignale durch Verringern und/oder Beseitigen der axialen Verschiebung ($\Delta t$), und
- Rekonstruieren mindestens eines zwei- oder dreidimensionalen Bildes des Objekts auf der Grundlage der korrigierten Detektionssignale.

**Revendications**

1. Système d'imagerie opto-acoustique comprenant

une unité d'irradiation (2) configurée pour irradier un objet (4) comprenant un tissu biologique avec un rayonnement électromagnétique,
une unité de détection (3) configurée pour détecter des ondes acoustiques générées dans l'objet (4) à une pluralité d'emplacements le long d'une dimension axiale (t) et le long d'au moins une dimension latérale (x, y), qui est perpendiculaire à la dimension axiale (t), en réponse à l'irradiation de l'objet avec un rayonnement électromagnétique et pour générer une pluralité de signaux de détection (I(x, y, t)) en conséquence, et une unité de traitement (7) configurée pour

- déterminer un déplacement axial (∆t) du cours d'un ou plusieurs premiers signaux de détection le long de la dimension axiale (t) par rapport au cours d'un ou plusieurs deuxièmes signaux de détection le long de la dimension axiale (t),
- corriger les signaux de détection par la réduction et/ou l'élimination du déplacement axial (∆t), et
- reconstruire au moins une image bidimensionnelle ou tridimensionnelle de l'objet sur la base des signaux de détection corrigés.

2. Système selon la revendication 1, l'unité de traitement étant configurée pour

- détecter une structure contenue dans les signaux de détection, la structure détectée correspondant à une représentation d'une composante de l'objet dans les signaux de détection, et
- déterminer le déplacement axial (∆t) du cours des premiers signaux de détection par rapport au cours des deuxièmes signaux de détection sur la base d'un décalage axial d'une section de la structure détectée par rapport à une structure attendue, qui correspond à la composante de l'objet, le long de la dimension axiale (t).

3. Système selon la revendication 2, la structure détectée correspondant à une représentation d'une composante endogène du tissu.

4. Système selon la revendication 3, la composante endogène du tissu étant au moins l'une parmi ce qui suit : au moins une couche contenant de la mélanine de tissu cutané, la couche basale (stratum basale) de l'épiderme de tissu cutané, la couche cornée (stratum corneum) de l'épiderme de tissu cutané, une couche de vaisseaux sanguins du tissu.

5. Système selon l'une quelconque des revendications 2 à 4, la structure détectée correspondant à une représentation d'une couche exogène configurée pour absorber au moins une partie du rayonnement électromagnétique.

6. Système selon la revendication 5, la couche exogène correspondant à un élément couplant disposé entre le tissu et l'unité de détection et configuré pour coupler acoustiquement l'unité de détection au tissu et/ou à un milieu appliqué à la surface du tissu, en particulier à la peau.

7. Système selon l'une quelconque des revendications 2 à 6, l'unité de traitement étant configurée pour améliorer et/ou détecter la structure contenue dans les signaux de détection par l'application d'un filtre passe-bande aux signaux de détection et/ou une segmentation d'informations contenues dans les signaux de détection.

8. Système selon l'une quelconque des revendications 2 à 7, l'unité de traitement étant configurée pour déterminer la section de la structure détectée, qui est décalée par rapport à la structure attendue le long de la dimension axiale (t) sur la base d'une segmentation, de préférence grossière, d'informations contenues dans les signaux de détection le long de la dimension axiale (t) et le long d'au moins une dimension latérale (x, y).

9. Système selon l'une quelconque des revendications 2 à 8, l'unité de traitement étant configurée pour déterminer une cartographie de la structure détectée le long de l'au moins une dimension latérale (x, y) et pour déterminer une cartographie de la structure attendue le long de l'au moins une dimension latérale (x, y) sur la base de la cartographie de la structure détectée.

10. Système selon la revendication 9, l'unité de traitement étant configurée pour déterminer la cartographie de la structure attendue par le lissage de la cartographie de la structure détectée.

11. Système selon la revendication 10, l'unité de traitement étant configurée pour lisser la cartographie de la structure détectée par l'application d'un filtre médian et/ou d'un filtre à moyenne mobile à la cartographie de la structure détectée le long d'au moins une dimension latérale (x, y).

12. Système selon l'une quelconque des revendications 9 à 11, l'unité de traitement étant configurée pour déterminer le décalage axial de la section de la structure détectée par rapport à la structure attendue le long de la dimension axiale (t) sur la base de la cartographie de la structure détectée et de la cartographie de la structure attendue.

**13.** Système selon l'une quelconque des revendications précédentes, l'unité de traitement étant configurée pour

- déterminer une fonction de corrélation croisée (ccorr($x_i$, $y_j$)), de préférence une fonction de corrélation croisée maximale cumulée, pour des signaux de détection le long de la dimension axiale (t), dans lequel la fonction de corrélation croisée (ccorr ($x_i$, $y_j$)) porte sur la corrélation croisée entre les signaux de détection le long de la dimension axiale (t), et
- déterminer le déplacement axial ($\Delta t$) du cours des premiers signaux de détection par rapport au cours des deuxièmes signaux de détection sur la base de la fonction de corrélation croisée (ccorr ($x_i$, $y_j$)).

**14.** Système selon la revendication 13, l'unité de traitement étant configurée pour déterminer le déplacement axial ($\Delta t$) du cours des premiers signaux de détection par rapport au cours des deuxièmes signaux de détection en outre sur la base d'une fonction de corrélation croisée lissée.

**15.** Système selon la revendication 14, l'unité de traitement étant configurée pour déterminer le déplacement axial ($\Delta t$) du cours des premiers signaux de détection par rapport au cours des deuxièmes signaux de détection sur la base d'une différence entre la fonction de corrélation croisée (ccorr ($x_i$, $y_j$)) et la fonction de corrélation croisée lissée.

**16.** Système selon la revendication 15, l'unité de traitement étant configurée pour déterminer la fonction de corrélation croisée lissée par le lissage de la fonction de corrélation croisée (ccorr($x_i$, $y_j$)).

**17.** Système selon la revendication 16, l'unité de traitement étant configurée pour déterminer la fonction de corrélation croisée lissée par l'application d'un filtre à moyenne mobile et/ou d'un filtre médian à la fonction de corrélation croisée (ccorr($x_i$, $y_j$)).

**18.** Procédé de traitement de données d'imagerie opto-acoustique mis en œuvre sur une unité de traitement, le procédé comprenant les étapes suivantes :

- la récupération d'une pluralité de signaux de détection (I(x, y, t)) représentant des ondes acoustiques générées dans un objet (4) à une pluralité d'emplacements le long d'une dimension axiale (t) et le long d'au moins une dimension latérale (x, y), qui est perpendiculaire à la dimension axiale (t), en réponse à l'irradiation de l'objet (4) avec un rayonnement électromagnétique,
- la détermination d'un déplacement axial ($\Delta t$) du cours d'un ou plusieurs premiers signaux de détection le long de la dimension axiale (t) par rapport au cours d'un ou plusieurs deuxièmes signaux de détection le long de la dimension axiale (t),
- la correction des signaux de détection par la réduction et/ou l'élimination du déplacement axial ($\Delta t$), et
- la reconstruction d'au moins une image bidimensionnelle ou tridimensionnelle de l'objet sur la base des signaux de détection corrigés.

Fig. 1

Scale bars 500 μm

Fig. 2

EP 3 654 828 B1

Fig. 3

a

uncorrected         corrected

surface (mm)

fs-axis (mm)     ss-axis (mm)

b   uncorrected

1   2   3

corrected

1   2   3

Hb (a.u.)    HbO$_2$ (a.u.)    Melanin (a.u.)

c

uncorrected

corrected

uncorrected

corrected

$sO_2$ (%)

80

60

40

0.5    1    1.5

length (mm)

Fig. 4

EP 3 654 828 B1

Fig. 5

EP 3 654 828 B1

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016038021 A1

**Non-patent literature cited in the description**

- **SCHWARZ.** *Combined label-free optical and optoacoustic imaging of model organisms at mesoscopy and microscopy resolutions*

- **BAGHERINIA.** *Visualization of the microcirculatory network in skin by high frequency optoacoustic mesoscopy*